# EUROPEAN PATENT SPECIFICATION

(11) **EP 3 404 014 B1**
(45) Date of publication and mention of the grant of the patent: **04.08.2021**
(21) Application number: 17830622.1
(22) Date of filing: 11.01.2017
(51) Int. Cl.: A23L 5/00, A23L 33/16, C07C 55/22, C07C 51/41, B01F 7/00

(54) **PRODUCTION METHOD FOR GRANULES OF CALCIUM SALT, AND SAID GRANULES**
HERSTELLUNGSVERFAHREN FÜR GRANULATE AUS CALCIUMSALZ UND BESAGTE GRANULATE
PROCÉDÉ DE PRODUCTION DE GRANULÉS DE SEL DE CALCIUM ET DESDITS GRANULÉS

(30) Priority: 22.07.2016 JP 2016144870
(43) Date of publication of application: 21.11.2018
(73) Proprietor: Unical, Kawasaki-shi, Kanagawa 215-0001 (JP)
(72) Inventor: NISHIMURA, Masahiko, Kawasaki-shi Kanagawa 215-0001 (JP)
(74) Representative: Pfenning, Meinig & Partner mbB
(86) International application number: PCT/JP2017/000692
(87) International publication number: WO 2018/016100

(56) References cited:
- WO-A-87/05507
- WO-A1-2005/110594
- JP-A- H0 998 738
- JP-A- H06 178 644
- JP-A- H08 157 380
- JP-A- 2016 016 176
- JP-A- 2017 048 154
- US-A- 5 260 082

## Description

### BACKGROUND

### 1. TECHNICAL FIELD

The present invention relates to a method of producing a calcium citrate granule and the granule.

### 2. RELATED ART

Calcium, which is an essential element for living bodies, is a substance essential for human bodies as it forms bones and teeth in human bodies and also regulate physiological functions in the bodies. Calcium is abundantly present in dairy products such as milk and cheese and also in seaweeds, small fish and sesames. Human beings take calcium from these foods. However, according to a report issued by the Ministry of Health, Labour and Welfare, although the recommended daily intake of calcium for adults is approximately 600 mg to 800 mg, the Japanese people take 531 mg of calcium per day on average, which is significantly short of the recommended intake, due to the changes in diet. In human bodies, the lack of calcium can trigger illness, for example, cause osteoporosis.

As disclosed in Patent Document 1, the present inventor found that the calcium compound obtained by baking external skeletons of sea urchin was excellently absorbed in the bodies and had excellent taste and flavor when consumed. In Patent Document 1, the present inventor also reports that the thus obtained calcium compound can allow a significantly increased amount of calcium to be absorbed in the bodies when mixed with chondroitin sulfate, which belongs to sulfomucopolysaccharides. In addition, the present inventor found in Patent Document 2 that the addition of chondroitin sulfate increases the amount of calcium absorbed in the bodies not only in the case of calcium derived from sea urchins but also in the case of organic carboxylate calcium salt. In this way, the present inventor has found that the amount of absorbed calcium is highly dependent on the concentration of chondroitin sulfate.

Patent document 1: Japanese Patent No. 2660906
Patent document 2: Japanese Patent No. 3131385

As a further example, document US 5 260 082 A describes a baked good which comprises a baked flour-containing mixture selected from the group consisting of dough and batters incorporated therein about 0.2 to about 5 weight percent of said baked good, a reaction product of a calcium compound selected from the group consisting of calcium hydroxide, calcium oxide and calcium carbonate with citric acid therein said reaction product has a mole ratio of calcium to citric acid from 2.5:2 to 2.95:2 and a pH value in a 1% water slurry of said reaction product from about 4 to about 7 at 25 DEG C.

Moreover, document WO 87/05507 A describes a calcium citrate composition having a bulk density greater than about 1.1 g/cc. Citric acid and a calcium compound are mixed to produce a mixture having a calcium compound/citric acid molar ratio of about 1.5. A hydrated mixture percent is produced by agitatively adding water to the mixture. The calcium compound, citric acid and water may also be mixed in one step. The hydrated mixture is blended to facilitate the reaction of citric acid with the calcium compound and to form a granulated mass primarily consisting of granules with diameters between about 1/64 inch (39.7 mm) and about 1/16 inch (159.0 mm). The granulated mass is then dried to a moisture content of between about 10 weight percent and about 13 weight percent to produce a calcium citrate composition having a bulk density greater than about 1.1 g/cc. For calcium citrate tablets this calcium citrate composition is formed into a tableting composition by subjoining one or more tableting binders. The tableting composition is then fed through a multiple-station tablet press to form high density calcium citrate tablets. The high density calcium citrate tablets are greater than about 15 weight percent calcium and have a calcium/citrate molar ratio of about 1.5. Such tablets characteristically have a density greater than about 1.5 g/cc and may for aesthetic or other purposes, be coated with mixtures comprising substances such as sugar, polyvinylpyrrolidone, calcium carbonate and titanium dioxide.

### SUMMARY OF THE INVENTION

### PROBLEMS TO BE SOLVED BY THE INVENTION

The readily absorbable calcium compositions disclosed in Patent Documents 1 and 2 are insoluble in water and have an average particle diameter of approximately 80 µm and a maximum particle diameter of approximately several hundred µm. Therefore, they can be successfully added to, for example, solid or semisolid food but may quickly settle out when added to beverage. This poses a problem.

In the conventional art, it is known to reduce the particle diameter by wet grinding, which has been used to reduce the particle size of calcium compositions.
A desired small particle size, however, requires cycle operation of wet medium grinding, which disadvantageously requires time and cost.

### SUMMARY

The invention is defined in the independent claims. A first embodiment of the present invention provides a method of producing a calcium citrate granule. The producing method includes stirring a hydrated lime slurry with 10 m/s or more of a circumferential velocity of a stir vane in a stirrer, dripping a carboxylic acid aqueous solution onto the slurry while the slurry is being stirred in the stirrer to produce a calcium salt, and spray-drying the calcium salt. The method further comprises, before the dripping, adding a dispersing agent including cyclodextrin to the slurry. The amount of the added dispersing agent is 5 weight parts to 20 weight parts on the basis of 100 weight parts of a calcium citrate solid content.

A second embodiment of the present invention provides a granule of a calcium citrate produced by the method of the invention, in which the granule is a secondary particle having an average particle diameter of 50 µm or less. Flat primary particles of the calcium citrate having an average length of 2 µm or less in a flat direction have aggregated into the secondary particle. The granule includes cyclodextrin as a binder.

### EFFECTS OF THE INVENTION

The present invention can produce calcium salt primary particle having a small size. Since smaller primary particles can readily disperse within solutions, the present invention can prevent calcium salt from forming a precipitate. A composition or granule of the readily dispersible calcium salt composition can be added not only to solid or semisolid food but also to beverage without causing dissociation of the component and uncomfortable coarse texture (roughness). Providing the readily dispersible calcium salt composition in the granular state can significantly reduce the cost of storage and transportation. As a secondary effect, addition of an auxiliary source material can prepare a readily dispersible calcium salt powder with improved calcium absorption in human bodies.

The invention is defined by the appended claims.

### BRIEF DESCRIPTION OF THE DRAWINGS

Fig. 1 is a schematic view showing primary and secondary particles of calcium citrate.
Fig. 2 generally shows an exemplary flow of steps of a method of producing a readily dispersible calcium salt.
Fig. 3 is a schematic view showing a stirrer with a stir vane.
Fig. 4A is an electron microscope photo showing a readily dispersible calcium salt powder enlarged 300 times.
Fig. 4B is an electron microscope photo showing a readily dispersible calcium salt powder enlarged 6000 times.

### (General Disclosure)

A method of producing a calcium salt granule may include stirring a hydrated lime slurry in a stirrer, may include dripping a carboxylic acid aqueous solution onto the slurry while the slurry is being stirred in the stirrer to produce a calcium salt, and includes spray-drying the calcium salt. The producing method may further include, before the spray-drying, subjecting the calcium salt to wet grinding.

In the method of producing a calcium salt granule, during the dripping, the carboxylic acid aqueous solution may be dripped from directly above a stir vane of the stirrer.

The producing method further includes, before the dripping, adding a dispersing agent to the slurry. In the method of producing a calcium salt granule, an amount of the added dispersing agent is 5 weight parts to 20 weight parts on the basis of 100 weight parts of a calcium salt solid content, the dispersing agent includes cyclodextrin, and the carboxylic acid aqueous solution includes a citric acid aqueous solution.

A granule of a calcium salt produced by the method as set forth above is a secondary particle having an average particle diameter of 50 µm or less, and flat primary particles of the calcium salt having an average length of 2 µm or less in a flat direction have aggregated into the secondary particle. Regarding the granule, the calcium salt includes calcium citrate and the granule includes cyclodextrin as a binder.

### DESCRIPTION OF EXEMPLARY EMBODIMENTS

Hereinafter, some embodiments of the present invention will be described.

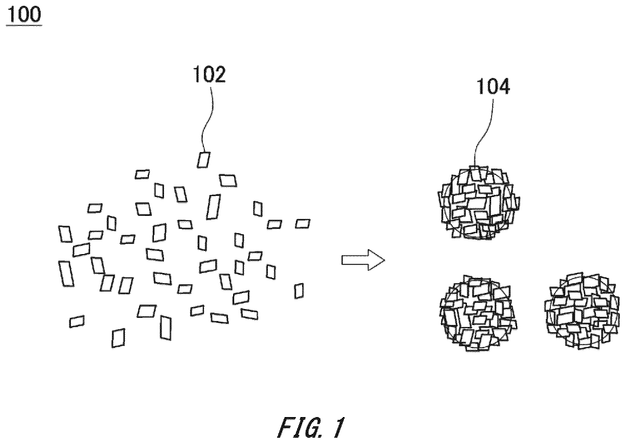

Fig. 1 is a schematic view showing particles 100 of calcium citrate, which is described later. Calcium citrate granules are produced in the following manner. The crystal nuclei are first formed and then grown to obtain a slurry containing primary particles 102 that are flat and plate-like crystals. The slurry containing the primary particles 102 is then spray-dried so that the primary particles aggregate to form secondary particles 104. In relation to this calcium salt, the present inventor has found that, once large plate-like crystals are formed, the calcium salt composition becomes a gel to turn into a slurry of high viscosity or become a solid, which makes it difficult to apply the spray-dry method.

In order to solve the above-described problem, the present inventor has found the following and completed the present invention. Instead of using the conventional method employing a homogeneous solution system, where a hydrated lime slurry is gradually added to a carboxylic acid aqueous solution, a producing method employing a heterogeneous system is used, where a carboxylic acid aqueous solution is gradually added to a hydrated lime slurry that is being violently stirred. In this manner, more microscopic crystals can be obtained and used as the calcium salt primary particles. The reason why such more microscopic crystals can be obtained has not been clarified, but the crystal growth rate of the calcium salt may be able to be reduced since the amount of the free calcium ions fed to form the salt is limited due to the fact that the hydrated lime slurry does not have a high solubility.

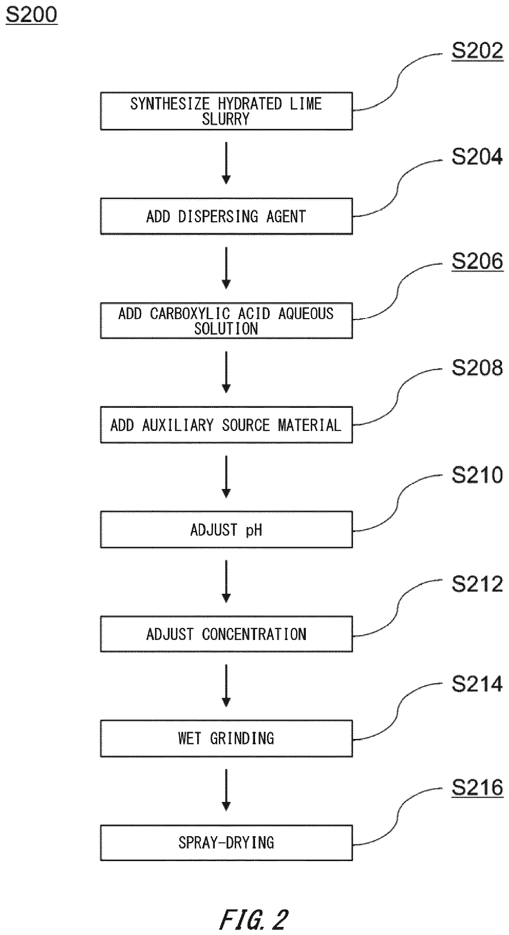

Fig. 2 generally shows an exemplary method S200 of producing a readily dispersible calcium salt granule relating to an embodiment of the present invention. The readily dispersible calcium salt producing method mainly includes a step S202 of preparing a hydrated lime slurry, a step S206 of adding a carboxylic acid aqueous solution and a step S216 of spray-drying. In addition, the producing method includes a step S204 of adding a dispersing agent for the purpose of obtaining more microscopic crystals of the carboxylic acid. For the purposes of making an readily absorbable calcium salt, the producing method may include a step S208 of adding an auxiliary source material that mainly contains chondroitin sulfate.
Furthermore, the producing method may additionally include a step S210 of adjusting the pH for the purposes of giving excellent flavor and mouthfeel to the beverage and food containing the above-described calcium salt. In order to produce granules of an appropriate size by the spray-drying step S216, the producing method may preferably include a step S212 of adjusting the concentration, which intends to adjust the viscosity. Furthermore, the producing method may include a step S214 of wet grinding for the purposes of obtaining primary particles of a more uniform size and stabilizing the quality of the calcium salt composition.

A readily dispersible calcium salt of the best embodiment contains citric acid as the carboxylic acid and cyclodextrin as the dispersing agent. In addition, the readily dispersible calcium salt is supplemented with an auxiliary source material, which is chondroitin sulfate, in order to make the calcium salt more readily absorbable.
The following describes the respective source materials in detail.

The carboxylic acid used in the present embodiment includes various options such as malic acid, but citric acid is especially preferred. Calcium citrate, which is produced as a result of the chelate bond between citric acid and calcium, is widely used as a food additive mainly for the purposes of adding calcium to food. Calcium forms chelate bond with citric acid and is then absorbed in the small intestine in living bodies. Therefore, compared with other organic carboxylates and inorganic salts, citric acid can achieve improved calcium absorption in living bodies.

The present inventor has found that, if more microscopic primary particles are obtained in the step of producing the calcium citrate, the calcium citrate can achieve significantly improved water dispersibility and is more unlikely to settle out within an aqueous solution. In order to improve the water dispersibility of calcium citrate, the primary particles have a horizontal size of 2 µm or less, more preferably, 1 µm or less. The secondary particles or granules have a particle diameter of 50 µm or less.

The dispersing agent is generally used to cause water insoluble or poorly-soluble fine particles to disperse in water. In the present embodiment, the dispersing agent could be water soluble cellulose and the like but is cyclodextrin. Cyclodextrin is one type of cyclic oligosaccharide, which has a cyclic structure where multiple glucose molecules are combined with each other through glucoside bond. For example, α-cyclodextrin containing 6 combined glucose molecules, β-cyclodextrin containing 7 combined glucose molecules, γ-cyclodextrin containing 8 combined glucose molecules and the like are well known.

Referring to the calcium citrate relating to the present embodiment, more microscopic primary particles can be obtained by adding cyclodextrin before the addition of the citric acid aqueous solution. Although the present invention does not desire to be limited by any theories, the above-mentioned microscopic primary particles can be obtained since, during the growth of the calcium citrate crystals following the formation of the crystal nuclei, the crystallization rate is reduced by cyclic compound such as cyclodextrin surrounding the crystal nuclei, which sterically hinders the crystallization.

Furthermore, cyclodextrin can prevent calcium citrate from aggregating during the process of wet grinding. Additionally, cyclodextrin serves as a binder when calcium salt primary particles aggregate to form secondary particles. To be specific, cyclodextrin dissolves when calcium salt granules are added to water. The disappearance of the binder facilitate the breakdown of the secondary particles, which can encourage the primary particles to disperse within the water.

In the present embodiment, 5 to 20 weight parts of cyclodextrin, described above, based on 100 weight parts of calcium salt solid content, in other words, cyclodextrin by 5 to 20% by weight, are added. If cyclodextrin is added by less than 5%, the calcium citrate primary particles have a horizontal size of larger than 2 µm, and the spray-drying step does not produce calcium salt granules but only produces granules of irregular shapes. Furthermore, when the producing method includes the wet grinding step, cyclodextrin poorly prevents the calcium citrate from aggregating during the wet grinding step if added by less than 5%. Thus, the wet grinding step cannot be performed. On the other hand, addition of cyclodextrin by more than 20% can no longer improve the effects of preventing the aggregation. The addition disadvantageously increases the water viscosity and requires increased force for the wet grinding step. Furthermore, since cyclodextrin is expensive, the production cost increases.

In the present embodiment, an auxiliary source material may be added to improve the absorption of calcium in living bodies. Chondroitin sulfate is particularly preferable as the auxiliary source material to achieve this purpose. Chondroitin sulfate is one type of mucopolysaccharides found in animal bodies, in which sulfuric acid is ester-linked to reiteratedly bonded disaccharides in which D-glucuronic acid is coupled in β-glycoside-linkage. As has been revealed by the present inventor in Patent Document 2, addition of chondroitin sulfate to organic carboxylate calcium salt enormously facilitate the absorption of calcium in living bodies. As disclosed in Patent Document 2, it is preferable to add 0.2 to 5% by weight, preferably 0.3 to 2.5% by weight, of chondroitin sulfate on the basis of the calcium content of calcium citrate since the absorption of calcium in living bodies is significantly improved.

In the present embodiment, commercially available quicklime can be directly used to prepare the hydrated lime slurry provided that it does not contain inorganics such as metals poisonous to human bodies. However, since the readily dispersible calcium salt according to the present invention is added to beverage and consumed by human bodies, it is preferable to use quicklime obtained by baking natural calcium sources, for example, external skeletons of sea urchins, seashells, egg shells and the like. Such calcium sources are normally disposed of as industrial wastes and easily available from the economical aspect.

The following describes the method of producing calcium salt granules and the production of the granules step-by-step with reference to Fig. 2. The following description is made taking calcium citrate containing cyclodextrin and chondroitin sulfate as an example of the most preferable carboxylate in the present invention, but the effective scope of the present invention is not limited to this example in any aspect.

In the step S202 of preparing a hydrated lime slurry, quicklime (CaO) is mixed with water to prepare a hydrated lime slurry (Ca(OH)₂). Here, the mixing ratio of the quicklime to the water is preferably 5 to 20 parts of quicklime to 95 to 80 parts of water by weight, more preferably, 5 to 10 weight parts of quicklime to 95 to 90 weight parts of water.

In order to efficiently prepare the hydrated lime slurry, quicklime, which is the source material, may be processed into fine particles using dry grinding and the like. By processing quicklime into fine particles in advance, the reaction between water and quicklime can end within a shorter period of time. By processing quicklime into fine particles in advance, the reaction between the hydrated lime, described later, and citric acid can also end within a shorter period of time. If available, commercially available hydrated lime with high quality can be used as the source material.

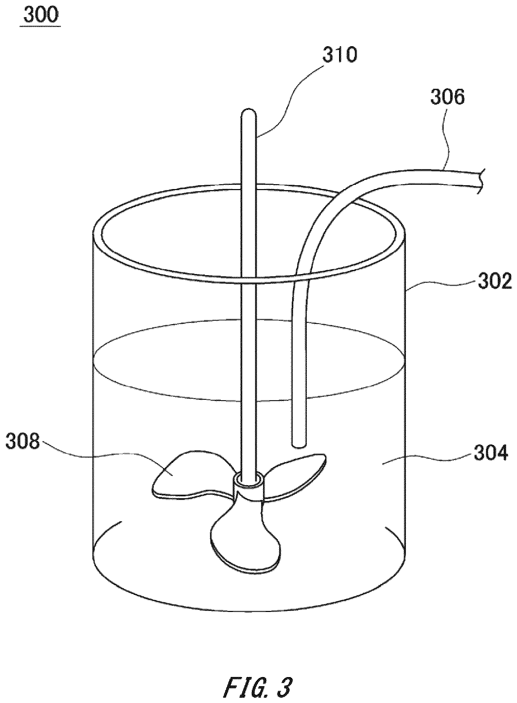

Fig. 3 is a schematic view showing a stirrer 300 with a stir vane. A hydrated lime slurry 304 is stirred in a reaction tank 302 by a stir vane 308, which is the mixer unit of the stirrer. A stir shaft 310 transfers the rotational energy generated by a motor and the like (not illustrated) to the stir vane so that the stir vane rotates. A carboxylic acid aqueous solution injection tube 306 is used to inject a citric acid aqueous solution, described later, from directly above the stir vane in order to mix the citric acid aqueous solution more homogenously.

The step S204 of adding the dispersing agent is performed to produce microscopic calcium citrate primary particles. Referring to the addition of the dispersing agent, cyclodextrin may be added in a powdery state, but may be preferably added in the state of being dissolved in water since cyclodextrin can homogenously disperse in the slurry. Since cyclodextrin also serves as a water-soluble binder, the bond between the calcium citrate and the binder is broken in the water, which can cause the calcium citrate primary particles to disperse in the liquid.

In the step S206 of adding the carboxylic acid aqueous solution, the citric acid aqueous solution is dripped into the violently stirred hydrated lime slurry in the reaction tank in order to synthesize calcium citrate. Note that it is preferable to drip the citric acid aqueous solution from directly above the stir vane of the stirrer since smaller citric acid crystals are produced. By violently stirring the slurry, smaller citric acid crystals are produced. Thus, the circumferential velocity of the stir vane is 10 m/s or more.

In the step S208 of adding the auxiliary source material, the auxiliary source material is added to the calcium citrate, which has been obtained as a result of adding the citric acid aqueous solution. It is preferable to add chondroitin sulfate in order to improve the absorption of calcium in living bodies. The amount of the added chondroitin sulfate is 0.2 to 5%, preferably 0.3 to 2.5%, by weight on the basis of the calcium content in the calcium citrate in the reaction liquid. In this way, calcium salt that achieves high calcium absorption in living bodies can be provided in the state of being dispersed in water.

In the step S210 of adjusting the pH, the pH of the calcium citrate is adjusted. Adjusting the pH is not an essential step to achieve microscopic calcium salt particles but can prevent the degradation of flavor and taste of the food and beverage added with the calcium salt composition. By adjusting the pH of the reaction liquid into the mildly acidic condition, the hydrated lime slurry and the citric acid can completely react with each other. For the reasons stated above, it is preferable to include the step of adjusting the pH. As the pH adjuster, citric acid, which is also the source material, can be used. It is preferable to add citric acid until the reaction liquid has pH 5 to 6 approximately, more preferably, pH 5.6.

In the step S212 of adjusting the concentration, the solid content concentration of the calcium citrate slurry is adjusted to be 10 to 20 weight% by adding purified water. This step can prevent an excessive increase in the viscosity of the calcium citrate slurry and gelation of the calcium citrate slurry in the steps of wet grinding and spray-drying, which are described later.

In the step S214 of wet grinding, calcium citrate in the liquid is subjected to wet grinding. The above-described steps of the producing method produce sufficiently microscopic calcium citrate primary particles and the calcium citrate granules are readily dispersible. This wet grinding step, however, contributes to stabilize the quality of the calcium salt composition and is thus preferably added. To be specific, the source material may adhere to the reaction tank and the like over time and get mixed in the calcium salt solution in the agglomerated state. By grinding the agglomerated source material, the calcium salt composition can be kept homogeneous. In addition, if large primary particles may be incidentally produced, such large primary particles are ground. In this way, more homogeneous products can be obtained. The wet grinding is not limited to any particular methods, but a medium-type ultra-fine grinding mill can be used, for example.

In the step S216 of spray-drying, the calcium salt solution is spray-dried into powders so that the readily dispersible calcium salt granules are produced. By adjusting the viscosity of the calcium salt solution to an appropriate level, the sprayed droplets can have an appropriate size and granules formed by powders having a particle diameter of 100 µm or less can be obtained. When particles having a particle diameter of 100 µm or more are added to beverage and the like to redisperse in the beverage, stirring needs to be performed for a very long period of time to successfully complete the redispersion. Such particles do not redisperse and precipitate if the stirring is performed only for a short period of time.

Spray-dryers are classified into several types including the disk atomizer type, the high pressure nozzle type, the double fluid nozzle type and the like depending on how the liquid is sprayed. The spray-dryer used in the spray-dry step is not limited to any particular type, and the spray dryer of any type can be used as long as particles having a maximum particle diameter of 100 µm or less can be obtained at a reasonable cost. If a spray dryer of the disk atomizer type, which is highly versatile, is used for the spray-dry step, the high-speed rotating type needs to be used. Otherwise, many of the produced particles have a particle size of 100 µm or more and post-processing is required to filter out the large particles and the like. Therefore, it is more preferable to use the double fluid nozzle type, which can produce more microscopic dry powders.

Having the above-described features, the present invention can provide a method of producing a calcium salt granule that can readily disperse when mixed with water and the granule. The present invention is further illustrated by referring to the following non-limiting working examples.

### (First Working Example)

Two hundred parts of purified water was poured into the reaction tank 300, and 20 parts of calcium produced by baking external skeletons of sea urchins was gradually added. In this way, a hydrated lime slurry was prepared. Seven parts of cyclodextrin (available under the trade name "dexipearl K-100" from Ensuiko Sugar Refining Co., Ltd.), in the state of being dissolved in 20 parts of purified water, was added to the hydrated lime slurry in the reaction tank and mixed by stirring. In another stirring-type reaction vessel, 45 parts of citric acid (available from Kenko Corporation) was gradually added to 100 parts of purified water. In this way, a citric acid aqueous solution was prepared. The citric acid aqueous solution was fed through the carboxylic acid aqueous solution injection tube 306 and gradually dripped from directly above the stir vane 308, which was violently stirring the hydrated lime slurry. After this, 0.25 parts of chondroitin sulfate (available from YAEGAKI Bio-industry, Inc.) was added to and mixed with the resulting slurry. Subsequently, citric acid was added until the pH value reached the predetermined value of 5.6. In this way, the calcium citrate slurry was synthesized.

Eighty parts of purified water was added to the calcium citrate slurry, which was then subjected to wet grinding process for a very short period of time. Following this, the spray-dry process was performed using a spray dryer (available under the trade name "Centri Dry Mill CDM10-550S" from MicroPowtec Co., Ltd.) under such conditions that the air pressure was 0.2 MPa and the spray amount was 15 L/h. In this way, calcium salt powders were produced.

### (Second Working Example)

A calcium citrate slurry was synthesized using calcium obtained by baking scallop shells. After this, the slurry was subjected to wet grinding and spray-drying to obtain calcium salt powders. The source materials that are to be mixed with each other, the mixing ratios, and the stir velocity are shown in Table 1-1 under the label of "2WE" short for SECOND WORKING EXAMPLE. The production procedures and techniques are the same as in the first working example.

### (First Comparative Example - not forming part of the invention)

One hundred parts of purified water was poured into the reaction tank 300 and 45 parts of citric acid was gradually added. In this manner, a citric acid aqueous solution was prepared. In another stirring-type vessel, 20 parts of calcium that was obtained by baking scallop shells was gradually added to 200 parts of purified water.

In this way, a hydrated lime slurry was prepared. The hydrated lime slurry was dripped onto the liquid surface of the citric acid aqueous solution in the reaction tank and mixed with the citric acid aqueous solution by stirring. Furthermore, 7 parts of cyclodextrin in the state of being dissolved in 20 parts of purified water was further added and mixed. After this, 0.25 parts of chondroitin sulfate was added to and mixed with the resulting slurry. Subsequently, citric acid was added until the pH value reached the predetermined value of 5.6. In this way, the calcium citrate slurry was synthesized.

One hundred and forty parts of purified water was added to the calcium citrate slurry, which was then subjected to wet grinding. During the grinding process, however, the slurry experienced an increase in viscosity and turned into a gel after several hours. For this reason, the spray-dry step could not be performed and calcium salt powders could not be obtained in the first comparative example.

### (Second Comparative Example - not forming part of the invention)

Calcium obtained by baking external skeletons of sea urchins was used to synthesize a calcium citrate slurry. The source materials that are to be mixed with each other, the mixing ratios, and the stir velocity are shown in Table 1-1 under the label of "2CE" short for SECOND COMPARATIVE EXAMPLE. The production procedures and techniques are the same as in the first comparative example. In the second comparative example, the calcium citrate slurry also experienced an increase in viscosity during the wet grinding process and turned into a gel after several hours as in the first comparative example. Thus, the spray-dry step could not be performed. As a result, calcium salt powders could not be obtained in the second comparative example.

**Table 1-1**

| CONDITIONS | | | 1WE | 2WE | 1CE | 2CE |
|---|---|---|---|---|---|---|
| MATERIALS BAKED TO OBTAIN CALCIUM | | | SEA URCHIN EXTERNAL SKELETONS | SCALLOP SHELLS | SCALLOP SHELLS | SEA URCHIN EXTERNAL SKELETONS |
| MIXING RATIO | CALCIUM OBTAINED BY BAKING | | 20 | 20 | 20 | 20 |
| | CARBOXYLIC ACID | | 45 | 45 | 45 | 45 |
| | CYCLODEXTRIN (DISPERSING AGENT) | | 7 | 7 | 7 | 7 |
| | CHONDROITIN SULFATE | | 0.25 | 0.25 | 0.25 | 0.25 |
| | WATER | | 400 | 460 | 460 | 380 |
| CALCIUM SALT SOLID CONTENT CONCENTRATION (WEIGHT%) | | | 14.0% | 12.4% | 12.4% | 14.6% |
| RATIO OF ADDED DISPERSING AGENT | | | 10.8% | 10.8% | 10.8% | 10.8% |
| REACTION CONDITION | | METHOD | ADD CITRIC ACID SOLUTION TO HYDRATED LIME SLURRY | ADD CITRIC ACID SOLUTION TO HYDRATED LIME SLURRY | ADD HYDRATED LIME SLURRY TO CITRIC ACID SOLUTION | ADD HYDRATED LIME SLURRY TO CITRIC ACID SOLUTION |
| | | STIR VELOCITY (m/s) | 10 | 13 | 2 | 10 |
| WET GRINDING | | | MEDIA-TYPE GRIND MILL | MEDIA-TYPE GRIND MILL | VISCOSITY INCREASE DURING GRINDING | VISCOSITY INCREASE DURING GRINDING |
| STABILITY OF SLURRY OVER TIME | | | GOOD | GOOD | GEL AFTER SEVERAL HOURS | GEL AFTER SEVERAL HOURS |
| HORIZONTAL SIZE OF PRIMARY PARTICLE (*µ*m) | | | 0.8 | 0.7 | 2.5 | 2.1 |
| GRANULE SIZE | SHAPE | | SPHERICAL | SPHERICAL | SPRAY-DRYING NOT POSSIBLE DUE TO GELATION | SPRAY-DRYING NOT POSSIBLE DUE TO GELATION |
| | AVERAGE PARTICLE DIAMETER (*µ*m) | | 10 | 8 | | |
| | MAXIMUM PARTICLE DIAMETER (*µ*m) | | 30 | 20 | | |
| REDISPERSIBILITY | | | GOOD | GOOD | - | - |

**Table 1-2**

| CONDITIONS | | 3WE | 3CE | 4CE |
|---|---|---|---|---|
| MATERIALS BAKED TO OBTAIN CALCIUM | | SCALLOP SHELLS | SCALLOP SHELLS | SCALLOP SHELLS |
| MIXING RATIO | CALCIUM OBTAINED BY BAKING | 20 | 20 | 20 |
| | CARBOXYLIC ACID | 45 | 45 | 45 |
| | CYCLODEXTRIN (DISPERSING AGENT) | 12 | 3 | 12 |
| | CHONDROITIN SULFATE | 0.25 | 0.25 | 0.25 |
| | WATER | 320 | 460 | 280 |
| CALCIUM SALT SOLID CONTENT CONCENTRATION (WE I GHT%) | | 16.9% | 12.4% | 18.8% |
| RATIO OF ADDED DISPERSING AGENT | | 18.5% | 4.6% | 18.5% |
| REACTION CONDITION | METHOD | ADD CITRIC ACID SOLUTION TO HYDRATED LIME SLURRY | ADD CITRIC ACID SOLUTION TO HYDRATED LIME SLURRY | ADD CITRIC ACID SOLUTION TO HYDRATED LIME SLURRY |
| | STIR VELOCITY (m/s) | 13 | 10 | 13 |
| WET GRINDING | | - | - | - |
| STABILITY OF SLURRY OVER TIME | | GOOD | VISCOSITY INCREASE OVER TIME | VISCOSITY INCREASE OVER TIME |
| HORIZONTAL SIZE OF PRIMARY PARTICLE (*µ*m) | | 0.9 | 2.6 | 1.7 |
| GRANULE SIZE | SHAPE | SPHERICAL | SPHERICAL+IRREGULAR | SPHERICAL |
| | AVERAGE PARTICLE DIAMETER (*µ*m) | 12 | 8 | 40 |
| | MAXIMUM PARTICLE DIAMETER (*µ*m) | 40 | 30 | 120 |
| REDISPERSIBILITY | | GOOD | GOOD | PARTICLE PRECIPITATE |

The above-described two working examples indicate that calcium salt granules can be produced under the above-described conditions if the producing method provided by the present embodiment is employed. On the other hand, the above-described two comparative examples indicate that, if the conventional technique of adding a hydrated lime slurry to a citric acid aqueous solution is employed, the slurry turns into a gel and granules cannot be produced even if the mixing ratios of the source materials such as calcium obtained by baking are the same as in the above-described working examples. In addition, these features of the present embodiment are not achieved merely by the stir velocity. This is clear from the results of the second comparative example, where the stirring was performed at a similar stir velocity as in the above-described working examples. In the first and second working examples, external skeletons of sea urchins and scallop shells were respectively baked to obtain calcium. Thus, the results of the first and second working examples have proved that the above-described effects of the present embodiment are not produced only when a particular calcium source obtained by baking is used.

The present embodiment is characterized in that the primary particles of a calcium salt are reduced in size to obtain granules of the calcium salt that are readily dispersible in water. In order to clarify the effect of this characterization, the following test was conducted.

### (Redispersibility Test)

Zero point one grams of the calcium salt granules, which were obtained in the above-described first and second working examples, were added to 100 mL of purified water and the resulting solution was poured into a glass vessel. A magnetic stirrer was used to disperse the granules. In this way, the redispersibility in water was evaluated. The results indicated that the calcium powders of the both working examples excellently redispersed in water.

### (Observation of particles and granules using an electron microscope)

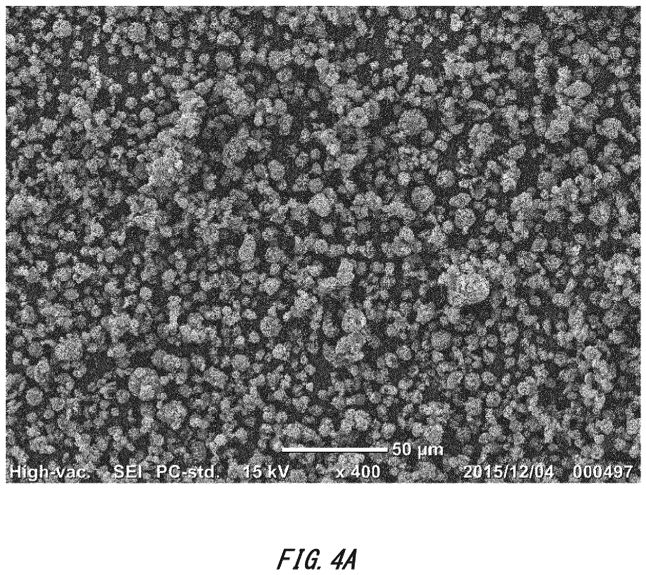

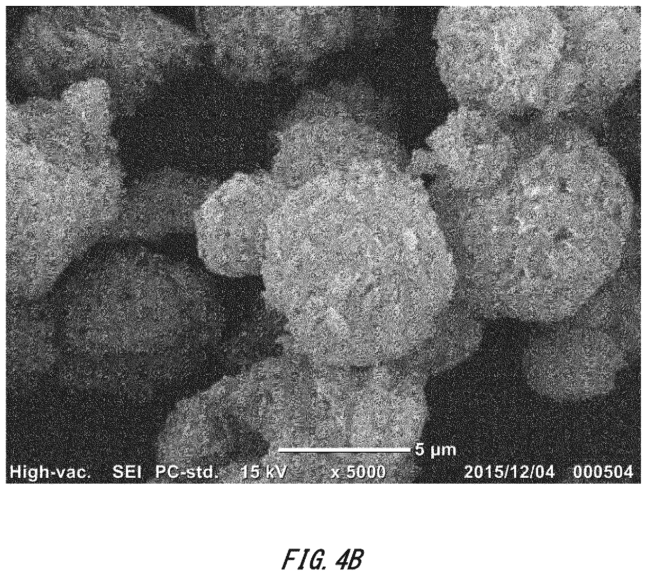

An electron microscope was used to observe the powders obtained by the above-described working examples. The observation revealed that flat calcium salt primary particles having an average length of 2 µm or less in the flat direction aggregated to form secondary particles having an average particle diameter of 50 µm or less. The observation using the electron microscope suggested that the cyclodextrin, which was added as a dispersing agent, served as a binder to assist the formation of the secondary particles. Figs. 4A and 4B are electron microscope photos showing such readily dispersible calcium salt powders, which are respectively enlarged 300 times and 6000 times. The slurries containing the primary particles of the first and second comparative examples were observed using the electron microscope. The observation confirmed that the primary particles have a significantly larger size than those obtained by the above-described working examples (not shown).

The present embodiment is characterized in that readily dispersible granules can be produced without performing wet grinding since calcium citrate primary particles have a sufficiently microscopic size and that cyclodextrin is preferably added by 5 to 20% on the basis of the calcium salt solid content. In addition, the present embodiment is also characterized in that the concentration of the calcium citrate slurry is preferably adjusted during the process of producing the calcium salt powders.
The following working examples were made in order to clarify such features and effects of the present embodiment.

### (Third Working Example)

Calcium obtained by baking scallop shells was used to synthesize a calcium citrate slurry. Differently from the first and second working examples, the slurry was spray-dried into calcium salt powders without performing the wet grinding step. The source materials that are to be mixed with each other, the mixing ratios, and the stir velocity of the third working example are shown in Table 1-2 under the label of "3WE" short for THIRD WORKING EXAMPLE. The production procedures and techniques of the third working example are the same as in the first working example except for that the wet grinding step is not performed.

### (Third and Fourth Comparative Examples)

Calcium obtained by baking scallop shells was used to synthesize calcium citrate slurries. The source materials that are to be mixed with each other, the mixing ratios, and the stir velocity are shown in Table 1-1 under the label of "3CE" short for THIRD COMPARATIVE EXAMPLE and "4CE" short for FOURTH COMPARATIVE EXAMPLE. The slurries of the third and fourth comparative examples both experienced an increase in viscosity over time, but the calcium citrate slurries of the third and fourth comparative examples could be both spray-dried into calcium salt powders without the wet grinding step. The production procedures and techniques of the third and fourth comparative examples are the same as in the first working example except for that the wet grinding step is not performed.

### (Redispersibility Test)

Referring to the granules of the calcium salts obtained in the third working example and the third and fourth comparative examples, their redispersibility in water was evaluated. The results showed that, while the calcium salt powders of the third working example and the third comparative example could excellently redisperse in water, the calcium salt powders of the fourth comparative example partly did not disperse and formed particles that precipitated in water.

### (Observation of particles and granules using an electron microscope)

The primary particles and granules of the third working example had a similar size as those of the first and second working examples, where the wet grinding step was included. On the other hand, the primary particles of the third comparative example had a significantly large horizontal size and the granules of the third comparative example had an irregular shape. Also in the fourth comparative example, the primary particles had a larger horizontal size than in the working examples, and, in particular, granules of a significantly large particle diameter were formed.

According to the above-described results of the third working example, it is clear that the present embodiment has such advantages that the wet grinding step does not need to be performed to obtain microscopic primary particles and granules of calcium salts and thus produce readily dispersible calcium salt powders.
In light of this, the wet grinding step may be included for the purposes of stabilizing the quality of the calcium salt composition.

When the wet grinding step was performed for a calcium citrate slurry that was prepared under similar source material mixing ratios as in the third comparative example, the calcium salt aggregated during the grinding step. Also taking into consideration that the calcium salt powders of the third comparative example have an irregular shape, this has confirmed that cyclodextrin should be preferably added by 5% or more. The fourth comparative example is different from the third working example only in that the calcium solid content has a higher concentration, but the calcium salt powders of the fourth comparative example was less readily-dispersible in water. For this reason, it is preferable to adjust the concentration of the solid content in the calcium citrate slurry before the wet grinding and spray-drying steps.

While the embodiments of the present invention have been described, the technical scope of the invention is not limited to the above described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order.

### EXPLANATION OF REFERENCE NUMERALS

100 : calcium citrate particle, 102 : primary particle, 104 : secondary particle, 200 : method of producing a readily dispersible calcium salt granule, 300 : stirrer, 302 : reaction tank, 304: hydrated lime slurry, 306 : carboxylic acid aqueous solution injection tube, 308 : stir vane, 310 : stir shaft

## Claims

1. A method of producing a calcium citrate granule, comprising:
stirring a hydrated lime slurry (102, 304) with 10 m/s or more of a circumferential velocity of a stir vane (308) in a stirrer (300);
dripping (S206) a citric acid aqueous solution onto the slurry while the slurry is being stirred in the stirrer to produce calcium citrate; and
spray-drying (S216) the calcium citrate, wherein
the method further comprises, before the dripping (S206), adding a dispersing agent (S204) including cyclodextrin to the slurry and wherein
the amount of the added dispersing agent is 5 weight parts to 20 weight parts on the basis of 100 weight parts of a calcium citrate solid content.

2. The method of producing a calcium citrate granule as set forth in Claim 1, wherein
during the dripping (S206), the citric acid aqueous solution is dripped from directly above the stir vane (308) of the stirrer (300).

3. A granule of a calcium citrate produced by the method as set forth in one of Claims 1 or 2, wherein
the granule (200) is a secondary particle (200) having an average particle diameter of 50 µm or less, wherein
flat primary particles (104) of the calcium citrate having an average length of 2 µm or less have aggregated into the secondary particle (200), and
the granule (200) includes cyclodextrin as a binder.

## Patentansprüche

1. Verfahren zur Herstellung eines Calciumcitratgranulatkorns, umfassend:
Rühren einer hydrierten Kalkmilchschlämme (102, 304) mit 10 m/s oder mehr einer Umfangsgeschwindigkeit eines Rührflügels (308) in einem Rührer (300);
Tropfen (S206) einer wässrigen Citronensäurelösung auf die Schlämme, während die Schlämme in dem Rührer gerührt wird, um Calciumcitrat zu produzieren; und
Sprühtrocknen (S216) des Calciumcitrats, wobei
das Verfahren ferner umfasst: vor dem Tropfen (S206), Hinzufügen eines Cyclodextrin enthaltenden Dispersionsmittels (S204) zu der Schlämme, und wobei
die Menge des hinzugefügten Dispersionsmittels 5 Gewichtsteile bis 20 Gewichtsteile auf der Basis von 100 Gewichtsteilen eines Calciumcitratfeststoffgehalts beträgt.

2. Verfahren zum Produzieren eines Calciumcitratgranulatkorns nach Anspruch 1, wobei
die wässrige Zitronensäurelösung während des Tropfens (S206) von direkt oberhalb des Rührflügels (308) des Rührers (300) getropft wird.

3. Granulatkorn eines Calciumcitrats, produziert nach dem Verfahren nach einem der Ansprüche 1 oder 2, wobei
das Granulatkorn (200) ein Sekundärpartikel (200) mit einem durchschnittlichen Partikeldurchmesser von 50 µm oder weniger ist, wobei
flache Primärpartikel (104) des Calciumcitrats mit einer Durchschnittslänge von 2 µm oder weniger in das Sekundärpartikel (200) aggregiert wurden und das Granulatkorn (200) Cyclodextrin als einen Binder umfasst.

## Revendications

1. Procédé de production d'un granulé de citrate de calcium, comprenant :
l'agitation d'une boue de chaux hydratée (102, 304) selon une vitesse circonférentielle d'une pale d'agitation (308) à l'intérieur d'un agitateur (300) de 10 m/s ou plus ;
le dégouttement (S206) d'une solution aqueuse d'acide citrique sur la boue tandis que la boue est en cours d'agitation à l'intérieur de l'agitateur afin de produire du citrate de calcium ; et
le séchage par pulvérisation (S216) du citrate de calcium ; dans lequel :
le procédé comprend en outre, avant le dégouttement (S206), l'ajout à la boue d'un agent de dispersion (S204) qui inclut de la cyclodextrine ; et dans lequel :
la quantité de l'agent de dispersion ajouté est de 5 parties en poids à 20 parties en poids sur la base de 100 parties en poids d'une teneur en solides de citrate de calcium.

2. Procédé de production d'un granulé de citrate de calcium tel que revendiqué selon la revendication 1, dans lequel :
pendant le dégouttement (S206), la solution aqueuse d'acide citrique est soumise à dégouttement depuis directement au-dessus de la pale d'agitateur (308) de l'agitateur (300).

3. Granulé d'un citrate de calcium produit au moyen du procédé tel que revendiqué selon l'une quelconque des revendications 1 ou 2, dans lequel :
le granulé (200) est une particule secondaire (200) qui présente un diamètre de particule moyen de 50 µm ou moins ; dans lequel :
des particules primaires plates (104) du citrate de calcium qui présentent une longueur moyenne de 2 µm ou moins se sont agrégées dans la particule secondaire (200) ; et
le granulé (200) inclut de la cyclodextrine en tant qu'agent de liaison.
